Europäisches Patentamt

European Patent Office

Office européen des brevets

(19)

(11) Veröffentlichungsnummer: **0 319 819**
**B1**

(12)                 **EUROPÄISCHE PATENTSCHRIFT**

(45) Veröffentlichungstag der Patentschrift: **23.01.91**

(21) Anmeldenummer: **88119788.3**

(22) Anmeldetag: **28.11.88**

(51) Int. Cl.⁵: **B 01 J 19/26,** B 05 B 7/04

(54) **Verfahren zur Herstellung von festen oder pastenförmigen Produkten.**

(30) Priorität: **07.12.87 DE 3741401**

(43) Veröffentlichungstag der Anmeldung:
**14.06.89 Patentblatt 89/24**

(45) Bekanntmachung des Hinweises auf die
Patenterteilung:
**23.01.91 Patentblatt 91/04**

(84) Benannte Vertragsstaaten:
**AT BE CH DE ES FR GB GR IT LI NL SE**

(56) Entgegenhaltungen:
**EP-A-0 032 341
EP-A-0 139 945
FR-A-2 141 490
FR-A-2 461 518**

(73) Patentinhaber: **Henkel Kommanditgesellschaft
auf Aktien
Postfach 1100 Henkelstrasse 67
D-4000 Düsseldorf 1 (DE)**

(72) Erfinder: **Blasey, Gerhard
Fürstenberger Strasse
D-4000 Düsseldorlf 13 (DE)**
Erfinder: **Breucker, Christoph
Unterdüssel 7
D-5608 Wülfrath (DE)**
Erfinder: **Gutsche, Bernhard, Dr.
Lessingstrasse 5 a
D-4010 Hilden (DE)**
Erfinder: **Jeromin, Lutz, Dr.
Am Bandsbusch 88
D-4010 Hilden (DE)**
Erfinder: **Panthel, Günther, Dr.
Am Bollenberger Busch 7
D-5657 Haan (DE)**
Erfinder: **Peukert, Eberhard
Dürerweg 15
D-4010 Hilden (DE)**
Erfinder: **Schmidt, Wolfgang
Sandstrasse 63
D-4019 Monheim (DE)**

## Beschreibung

Die Erfindung betrifft ein Verfahren zur Herstellung von festen oder pastenförmigen Produkten durch Umsetzung stöchiometrischer Mengen mehrerer Reaktanden in einer schnell ablaufenden Reaktion und Versprühen der Reaktionsprodukte in einer Sprüheinrichtung.

Typische Beispiele für schnell ablaufende chemische Reaktionen im Sinne der vorliegenden Erfindung sind die Neutralisation von Säuren, die Verseifung von Estern sowie Alkylierungsreaktionen, insbesondere Alkylierungen tertiären Amine.

Neutralisationsreaktionen von Sulfonsäuren oder Fettsäuren laufen spontan ab. Probleme bei der Reaktionsführung können dann entstehen, wenn die Reaktanten, d.h. Säuren und Laugen, nicht miteinander mischbar sind und/oder wenn das entstehend Neutralisationsprodukt ein fester oder pastenförmiger Stoff ist. In diesen Fällen wird die Güte der Neutralisation von der Intensität der Reaktandenvermischung und/oder der Durchmischung des Reaktionsgemisches bestimmt.

Die Neutralisation von Sulfonsäuren zu waschaktiven Sulfaten oder Sulfonaten erfolgt großtechnisch diskontinuierlich in Neutralisationskesseln oder kontinuierlich in Umwänzkreisläufen. Im letzteren Falle wird die Sulfonsäure zusammen mit der Lauge unter intensiver Vermischung durch Rührer, Zentrifugalpumpen und statische Mischelemente in bereits neutralisiertes Produkt eingegeben, wobei das Verhältnis von Kreislaufstrommenge zu frisch zudosierter Sulfonsäure und Lauge im Bereich von 10:1 bis 20:1 liegt. Bei der Neutralisation hydrolyseempfindlicher Produkte wie Schwefelsäurehalbestern von Fettalkoholen und Fettalkoholethern muß hierbei eine gleichmäßig gute Durch- und Vermischung des Neutralisationsgemisches gewährleistet sein, um Produktverluste auszuschließen. Zur Abfuhr der freiwerdenden Reaktionswärme sind bei diskontinuierlicher und kontinuierlicher Fahrweise Wärmeaustauschelemente notwendig, damit Produktüberhitzungen ausgeschlossen werden können.

Mit diesen Verfahren lassen sich Neutralisationsprodukte mit einem Anteil an waschaktiver Substanz (WAS) von ca. 60 Gew.-% herstellen. Der Hauptanteil der restlichen ca. 40 Gew.-% besteht aus Wasser, das bei der Einarbeitung der waschaktiven Sulfate/Sulfonate in pulverförmige Waschmittel z.B. durch Sprühtrocknung entfernt werden muß.

Eine Verminderung des Wassergehaltes zur Reduzierung des Energieaufwandes bei der Trocknung wäre wünschenswert und durch den Einsatz höher konzentrierter Laugen in der Neutralisationsreaktion auch realisierbar.

Einfache Stoffbilanzen zeigen, daß sich bei der Neutralisation von Sulfonsäuren mit 50 %-iger Natronlauge Neutralisationsprodukte mit einem Anteil an waschaktiver Substanz von ca. 80% und einem Wasseranteil von ca. 20% herstellen lassen. Die Energiebilanz der Neutralisationsreaktion liefert außerdem das Ergebnis, daß ca. 40—50% des während der Neutralisation gebildeten und des mit der 50%-igen Natronlauge in das Reaktionsgemisch eingebrachten Wassers mit der freiwerdenden Reaktionswärme verdampft werden kann. Theoretisch entstehen somit bei Einsatz von 50%-iger Natronlauge unter Ausnutzung der Reaktionswärme zur Trocknung Neutralisationsprodukte mit einem Anteil von ca. 90% waschaktiver Substanz und nur noch ca. 10% Wasser. Die Neutralisationsprodukte von Sulfonsäuren auf der Basis von Fettalkohol und Fettsäureester sind unter diesen Bedingungen bereits zu rieselfähigen Pulvern verarbeitbar. Durch Ausnutzung der Reaktionswärme können jedoch auch andere, unerwünschte flüchtige Substanzen aus den Produkten entfernt werden. Als Beispiel sie hier die Reduzierung des Dioxan-Gehaltes in Fettalkoholethersulfaten genannt.

Das vorstehend beschriebene, großtechnisch eingesetzte Verfahren zur Neutralisation von Sulfonsäuren läßt den Einsatz hochkonzentrierter Laugen aber nicht zu, da das Reaktions- bzw. Neutralisationsprodukt mit zunehmendem Gehalt an waschaktiver Substanz immer viskoser und schließlich pastenförmig wird. Ab einem Gehalt von ca. 60 Gew.-% waschaktiver Substanz sind Sulfate/Sulfonate kaum noch pumpbar, und eine gleichmäßige Ver- bzw. Durchmischung der Reaktionskomponenten bzw. des Reaktionsgemisches und die sichere Abfuhr der Reaktionswärme ist nicht mehr beherrschbar.

Vergleichbar mit der Neutralisation von Sulfonsäuren zu Herstellung waschaktiver Sulfate/Sulfonate ist die Herstellung von Seifen durch die Neutralisation von Fettsäuren mit Laugen. Hierbei wird der Neutralisationsvorgang jedoch nicht nur durch das pastöse Fließverhalten der während der Reaktion gebildeten Seife erschwert. Da die wäßrigen Laugen und die Fettsäuren nicht ineinander löslich sind, ist ein entsprechend hoher Dispergiergrad notwendig, um eine ausreichend große Stoffaustauschfläche zwischen den beiden flüssigen Phasen und damit eine genügend hohe Reaktionsgeschwindigkeit zu erzeugen. Die bisher üblichen Verfahren zur Herstellung von Grundseifen aus Fettsäuren lassen sich in zwei Verfahrensschritte unterteilen:

1. Zusammenführen, Homogenisieren oder Emulgieren der ineinander unlöslichen Reaktanden und Durchführung der Neutralisations- bzw. Verseifungsreaktion in Rührkesseln, Reaktionskolonnen oder Schlaufenreaktoren.

2. Mehrstufige Trocknung der Grundseite mit einem Aktivsubstanzgehalt von ca. 60% auf 80%.

Die Neutralisation von Fettsäuren mit Lauge ist wie die Neutralisation von Sulfonsäuren mit Laugen eine spontan ablaufende Reaktion, wenn es gelingt, die beiden ineinander unlöslichen Reaktanden ausreichend fein dispergiert zusammenzuführen. Die Reaktion kann bei Raumptemperatur gestartet werden, wobei eine kräftige Erwärmung des Reaktionsgemisches bzw. der Reaktionsprodukte eintritt.

Wie bei der Neutralisation von Sulfonsäuren zeigen die Stoff- und Energiebilanzen für die Fettsäureneutralisation, daß beim Einsatz von 50%-iger Natronlauge die Herstellung einer Grundseife mit einem Aktivsubstanzgehalt von ca. 80% und ca. 20% Wassergehalt in einem einzigen Verfahrensschritt möglich ist.

Anstelle von reinen Fettsäuren können auch Fettsäuremethylester durch Verseifung mit Natronlauge zu Seifen umgesetzt werden. Bei dieser Reaktion entsteht als Nebenprodukt Methanol, das aus der Grundseife bzw. Feinseife entfernt werden muß. Dies geschieht im allgemeinen durch Ausdampfen bzw. Strippen mit Dampf. Die angewandten Verfahren zur Herstellung von Seifen aus Methylestern entsprechen der Verfahren zur Herstellung von Seifen aus Fettsäuren, wobei eine zusätzliche Verfahrensstufe, das Entfernen von Methanol, notwendig ist. Im Unterschied zur Fettsäureneutralisation ist die Umsatzgeschwindigkeit der Methylesterverseifung stark von der Reaktionstemperatur abhängig. Während bei Raumtemperatur die Verseifungsreaktion nur langsam fortschreitet, wird sie durch höhere Temperaturen (z.B. 100°C) stark beschleunigt. Sie läuft praktisch momentan ab, wenn es bei 130—140°C gelingt, den Methylester und die Lauge fein dispergiert in Kontakt zu bringen.

Aus der EP—A 0 083 122 ist ein Verfahren der eingangs genannten Art bekannt, gemäß dem zwei Reaktandenströme, bestehend jeweils aus Säure und Lauge, und unabhängig davon ein Gasstrom einer Sprühdüse zugeführt werden; die Neutralisationsreaktion zwischen den versprühten Reaktanden erfolgt nach dem Verlassen der Düse. Das Massenverhältnis zwischen Gas und Reaktanden beträgt etwa 0,4. Das Verfahren kann jedoch nur innerhalb enger Verfahrensparameter betrieben werden, wenn ein hoher Neutralisationsgrad erreicht und ein Verstopfen der Sprühdüsen verhindert werden soll. Zudem erfordert das bekannte Verfahren kompliziert aufgebaute Düsen mit einer Vielzahl von konzentrisch angeordneten Kanälen für die getrennte Zufuhr von Reaktanden und gasförmigem Medium.

Die Erfindung ist auf ein Verfahren der eingangs genannten Art gerichtet, das Sprüheinrichtungen einfacher Konstruktion erfordert, mit über weite Bereiche variierbaren Produktionsleistungen betrieben werden kann (z.B. 1,5 bis 200 kg/h Produkt für eine der im Verfahren der Erfindung eingesetzten Düsen), und das wenig störungsanfällig ist, da ein Verstopfen der Sprüheinrichtung vermieden wird.

Diese Aufgabe wird erfindungsgemäß dadurch gelöst, daß man die getrennten Reaktandenströme, gegebenenfalls nach Erwärmen, jeweils mit einem gasförmigen Medium beaufschlagt, anschließend zusammenführt und schließlich durch die Sprüheinrichtung mit einer Geschwindigkeit von 0,1 bis 15 m/ sec, errechnet aus den gasfreien Reaktandenströmen und bezogen auf den freien Querschnitt der Sprüheinrichtung, preßt, wobei die Massen von Reaktanden und gasförmigem Medium so bemessen sind, daß sich ein Massenverhältnis von gasförmigem Medium zu Produktstrom zwischen 0,04 und 0,3 (kg/kg) ergibt.

Das Verfahren der Erfindung kann überall dort eingesetzt werden, wo im Verlauf schneller chemischer Reaktionen feste bzw. pastenförmige Produkte gebildet werden, z.B. bei der Neutralisation von Säuren, insbesondere Carbonsäure, Schwefelsäurehalbestern und Sulfonsäuren sowie bei der Verseifung von Estern, z.B. niedrig-Alkylestern von Fettsäuren, insbesondere zu den entsprechenden Alkalischen mittels konzentrierter wäßriger Alkalien. Weiterhin geeignet ist das Verfahren der Erfindung zur Herstellung von Alkalisalzen, insbesondere des Natriumsalzes, der Monochloressigsäure sowie zur Herstellung von quartären Ammoniumverbindungen durch Alkylierung tertiärer Amine, z.B. mit Alkylhalogeniden. Besonders bevorzugt ist der Einsatz des Verfahrens der Erfindung bei der Herstellung fester, rieselfähiger, waschaktiver Produkte.

Zu den erfindungsgemäß herstellbaren festen oder pastenförmigen waschaktiven Produkten gehören unter anderem die Neutralisationsprodukte von Alkylarylsulfonsäuren, α-Sulfofettsäureester und Fettsäuren sowie der Schwefelsäurehalbester von alkoxylierten, insbesondere ethoxylierten Fettalkoholen und Phenolen und der Sulfobernsteinsäure, weiterhin die Verseifungsprodukte von Fettsäureestern, insbesondere Fettsäuremethylester. Die Neutralisation bzw. Verseifung erfolgt bevorzugt mit konzentrierten wäßrigen Alkalien, z.B. mit etwa 50 Gew.-% NaOH enthalten der Natronlauge. Als gasförmiges Medium eignen sich insbesondere gegenüber den Reaktanden und Reaktionsprodukten inerte Gase wie Luft, Stickstoff und Wasserdampf. Wasserdampf wird insbesondere dann eingesetzt, wenn die Reaktandenströme oder einer der Reaktandenströme vor der Zusammenführung erwärmt werden sollen. Der Abstand des Ortes der Zusammenführung der gasbeaufschlagten Reaktandenströme von der Sprüheinrichtung ist von den Verfahrensbedingungen und Stoffsystemen abhängig und sollte möglichst klein gehalten werden. Er kann durch den Fachmann mittels einfacher Versuche ermittelt werden; dabei ist lediglich darauf zu achten, daß ein Verstopfen der Düse aufgrund zu früh einsetzender Neutralisations- bzw. Verseifungsreaktionen vermieden wird.

Unter wäßrigen Alkalien im Sinne der Erfindung sind insbesondere die Hydroxide, Carbonate und Hypochlorite des Natriums und Kaliums in wäßriger Lösung bzw. Suspension sowie deren Mischungen zu verstehen. Mit den Hypochloriten läßt sich gleichzeitig eine bei vielen Produkten erwünschte Bleichwirkung erzielen.

Die Obergrenze der Geschwindigkeit der zusammengeführten Reaktandenströme, bezogen auf den freien Querschnitt der Sprüheinrichtung, ergibt sich durch die Schallgeschwindigkeit des Gases und dem eingestellten Verhältnis Gasmassenstrom/Produktmassenstrom. Bei ihrer Berechnung werden die gasfreien Reaktandenströme zugrunde gelegt.

Durch die Zugabe eines gasförmigen Mediums in die Reaktandenströme vor deren Zusammenführung ist das Verfahren der Erfindung unabhängig vom Typ und von der Konstruktion der jeweils verwendeten Sprühdüsen; es ist sogar eine Sprühneutralisation bzw. Verseifung durch ein einfaches Rohr möglich. Zur Erreichung einer guten Vermischung und zum Erzielen eines vollständig neutralisierten, homogenen Produkts sind beim Versprühen mit einem Rohr natürlich höhere Massenverhältnisse zwischen gasförmigem Medium und Produktstrom notwendig als beim Einsatz von Düsen, die auch sonst zum Zerstäuben von Flüssigkeiten verwendet werden. Geeignet hierzu sind z.B. Hohlkegel-, Vollkegel-, und Flachstrahldüsen mit entsprechenden Drallkörpern oder Drallschlitzen bzw. mit einem tangentialen Zulauf in einem Drallraum.

Geeignete Düsentypen und Angaben zur Dimensionierung sind in der einschlägigen Literatur, z.B. in K. Chaster, Spray Drying Handbook, John Wiley & Sons, New York 1979, beschrieben.

Die Dimensionierung der Düsenöffnung ist abhängig vom gewünschten Durchsatz.

Das Verfahren der Erfindung ist insbesondere geeignet zur Herstellung fester, rieselförmiger, waschaktiver Produkte, vorzugsweise zur Herstellung von waschaktiven Alkalisulfaten bzw. -sulfonaten. Hierzu verwendet man vorzugsweise Reaktandenströme, insbesondere Säureströme, die eine Lösung bzw. Suspension von Puffersubstanzen enthalten. Die art der Puffersubstanzen ist dem Fachmann auf dem Gebiet hydrolysempfindlicher Tenside bekannt.

Gemäß einer weiteren vorteilhaften Ausführungsform der Erfindung setzt man zur Herstellung von Produkten, die zur Farbverbesserung gebleicht werden müssen, insbesondere Fettsäure(niedrig)alkylestersulfonaten, Natriumperborat als Puffersubstanz hinzu; diese Puffersubstanz wirkt gleichzeitig als Bleichmittel, so daß gegebenenfalls auf eine weitere Verfahrensstufe verziechtet werden kann.

Gemäß einer weiteren vorteilhaften Ausführungsform der Erfindung nutzt man die Neutralisationswärme bzw. die bei der Verseifung von Estern erforderliche, zuzuführende Energie zur Verdampfung des Reaktionswassers bzw. der meist über die wäßrigen Alkalien zugeführen Wassermenge, so daß man feste, rieselfähige waschaktive Produkte erhalten kann. Weiterhin kann die vorgenannte Neutralisationswärme bzw. Energie dazu benutzt werden, im Reaktionssystem enthaltene oder gebildete niedrig-siedende Lösemittel, z.B. Methanol, vollständig zu entfernen.

Ein besonders vorteilhaftes Merkmal des Verfahrens der Erfindung ist dadurch begründet, daß man bei der Herstellung von Fettalkohol-ethersulfaten durch Neutralisation von Schwefelsäurehalbestern von Fettalkoholethoxylaten mit wäßrigen Alkalien Produkte erhält, die—bezogen auf den Feststoffgehalt—Dioxangehalte unterhalb von 200 ppm aufweisen.

Das Verfharen der Erfindung ist weiterhin geeignet zur Herstellung von Alkalisalzen substituierter Fettsäuren durch Neutralisation oder von quartären Ammoniumverbindungen durch Alkylierung der entsprechenden tertiären Amine, insbesondere mit Alkylhalogeniden.

Durch das Verfahren der Erfindung erreicht man eine intensive Vermischung der Reaktanden während ihrer Zusammenführung und eine sofort im Anschluß an die Intensivmischung stattfindende Feinverteilung des Reaktionsgemisches durch Versprühen über eine Düse. Die Vermischung und Feinverzeilung kann durch die kinetische Energie der Reaktanden (Strömungsgeschwindigkeit) und durch die potentielle Energie des Reaktionsgemisches (Druck) erfolgen. Beide Vorgänge werden durch das zuzusetzende gasförmige Medium begünstigt. Die Zumischung des Mediums zu den Reaktanden vor deren Zusammenführung verhindert das Entstehen größerer Produkteinheiten bei dem Zusammentreffen; dadurch wird die notwendige Mikrovermischung der Reaktanden gesteiegert und der nachfolgende Sprühvorgang erleichtert.

Im Falle exothermer Reaktionen, wie z.B. bei der Neutralisation von Sulfonsäuren und Fettsäuren, dient das gasförmige Medium außer zur Intensivierung der Vermischung auch zur Abfuhr der Reaktionswärme und zur Herabsetzung der Reaktionstemperatur. Ist andererseits eine höhere Reaktionstemperatur erforderlich, um unter den Bedingungen eines Sprühvorganges zum Erzielen hoher Umsatzgrade die Reaktionsgeschwindigkeit zu erhöhen, kann zusätzlich zu den Reaktandenströmen auch das gasförmige Medium vorgewärmt und durch direkten Wärmeaustausch mit dem Reaktionsgemisch zur Einstellung der notwendigen Reaktionstemperatur eingesetzt werden. Als Beispiel hierfür ist die Hydrolyse von Fettsäuremethylestern mit konzentrierten wäßrigen Alkalien zu nennen. Die Zugabe des gasförmigen Mediums in die Reaktandenströme ermöglicht auch bei der Bildung pastöser oder fester Produkte ein Versprühen bei kleinen Drücken. Die Variation der Düsenleistung kann über einfache Änderungen der Volumenströme des gasförmigen Mediums erfolgen.

In der praktischen Durchführung des Verfahrens der Erfindung werden die in den entsprechenden Vorlagen bevorrateten Reaktanden untre Verwendung von Kolbenpumpen stöchiometrisch dosiert in eine nicht weiter modifizierte handelsübliche Düse bzw. ein Sprührohr gefahren. Vor dem Zusammentreffen der Reaktanden unmittelbar vor der Düse erfolgt das Zumischen des gasförmigen Mediums in die Reaktandenströme. Bei der Neutralisation von Sulfonsäuren und Fettsäuren verwendet man als gasförmiges Medium Luft bzw. Stickstoff bei Raumtemperatur. Die basische Hydrolyse des Methylesters kann beispielsweise methylesterseitig unter Einsatz von Stickstoff mit Raumtemperatur und alkaliseitig unter Einsatz von überhitztem Wasserdampf (150 bis 160°C) als gasförmiges Medium erfolgen.

Die Steuerung der Reaktionstemperatur kann direkt über die versprühten Massen- bzw. Volumenströme der Reaktanden und der Temperatur derselben geschehen. Zusätzlich ist eine Beeinflussung durch den Massen- bzw. Volumenstrom des gasförmigen Mediums und der Temperatur desselben möglich.

Eine Regelung des pH-Wertes des Produkts im technisch interessanten Bereich (pH 7 bis pH 9) wird durch die sehr geringen Mengen und Verweilzeiten der Reaktanden in der Düse und die starke Abhängigkeit des pH-Wertes von dem Massenverhältnis zwischen Säure und konzentrierten wäßrigen Alkalien erschwert. So führt z.B. ein geringfügiges Absinken der eingesetzten Menge an Alkylbenzolsulfonsäure um 1% zu einem Anstieg des pH-Wertes des Produkts von pH 4 auf pH 11.

Durch Zugabe von Puffern wie Zitronensäure und insbesondere Natriumperborat (Pufferwirkung der Borsäure) ergibt sich durch die Pufferwirkung eine deutliche Verflachung der Neutralisationskurve.

Wird beispeilsweise zu Alkylbenzolsulfonsäure 6% Natriumperborat zugegeben und als Suspension der Düse zugeführt, so bewirken Schwankungen der Reaktandenströme von 2% lediglich Veränderungen des pH-Wertes zwischen 7 und 9.

Vorteilhaft ist bei dem hier beschriebenen Verfahren das niedrige Massenverhältnis zwischen Hilfsstoff und Produkt. Zur Erreichung einer sehr guten Vermischung und zum Erzielen eines vollständig neutralisierten, homogenen Sprühprodukts sind Massenverhältnisse von 0,3 kg, insbesondere von weniger als 0,3 kg gasförmiges Medium pro kg Fertigprodukt bei der Herstellung eines vollständig neutralisierten Produkts bei der Neutralisation von Sulfonsäuren ausreichend. Die nachfolgende Tabelle 1 zeigt die Betriebsbedingungen beim Einsatz unterschiedlicher Düsentypen und Düsendurchmessern in der Neutralisation von Alkylenzolsulfonsäure mit 50 %-iger Natriumhydroxidlösung.

## TABELLE 1
Betriebsbedingungen bei der Neutralisation von Alkylbenzolsulfonsäure

| Düsentyp | Bohrungsdurchmssser [mm] | Druck vor der Düse [bar] | Produktgesch- windigkeit [m/s]* | Verhältnis Gas-/Produkt- Massenstrom (kg/kg) |
|---|---|---|---|---|
| 30°-Hohl-Kegeldüse | 1,2 | 8,8 | 7,1 | 0.08 |
| (Schlick, Modell 100/200) | 1,2 | 10,7 | 15,0 | 0,07 |
| | 2,7 | 2,8 | 1,4 | 0,10 |
| | 2,7 | 6,9 | 6,9 | 0,05 |
| | 3,4 | 2,8 | 0,9 | 0,09 |
| | 3,4 | 5,7 | 4,4 | 0,04 |
| | 5,0 | 3,1 | 1,1 | 0,08 |
| | 5,0 | 4,1 | 2,0 | 0,04 |
| Rohr | 3,0 | 6,9 | 1,5 | 0,20 |
| | 3,0 | 9,7 | 2,7 | 0,19 |
| | 6,0 | 2,7 | 0,4 | 0,23 |
| | 6,0 | 4,9 | 1,4 | 0,15 |
| Ringspalt (Schlick, | $d_a$=5,0 $d_i$=3,0 | 5,5 | 0,8 | 0,29 |
| Modell 942) | $d_a$=5,0 $d_i$=3,0 | 9,4 | 3,1 | 0,13 |

*Bezogen auf den freien Querschnitt der Düsenöffnung

Das Verfahren der Erfindung wird im folgenden anhand bevorzugter Ausführungsbeispiele näher erläutert. Zum Einsatz kam eine Düse vom Typ Schlick, Modell 942, mit den Dimensionen gemäß Tabelle 1.

Beispiel 1
Sprühneutralisation von Alkylbenzolsäure (ABS)

Einsatz:
ABS (Basis $C_{12/14}$), $\dot{m}$ ca. 60 kg/h, t ca. 60°C
Natronlauge mit 50 Gew.-% NaOH, t ca. 40°C
Sprühdruck: 4,5 bar
$N_2$-Bedarf: 2 $m^3$/h

Sprühprodukt (Angaben in Gew.-%):

|          |      |
|----------|------|
| WAS:     | 84,8 |
| US:      | 1,6  |
| $Na_2SO_4$: | 0,8 |
| $H_2O$:  | 11,5 |
| pH-Wert: | 9,6  |

(1 %-ige Lösung, bez. auf WAS)

Farbenzahl n. Klett: 59

(5 %-ige Lösung, bez. auf WAS, 4 cm Küvette, Blaufilter Nr. 42)

Beispiel 2

Sprühneutralisation von Fettalkoholsulfonsäure (FAS)

Einsatz:
FAS (Basis $C_{12/16}$), ṁ ca. 60 kg/h, t ca. 60°C
Natronlauge mit 50 Gew.-% NaOH, t ca. 40°C
Sprühdruck: 4,5 bar
$N_2$-Bedarf: 2 $m^3$/h

Sprühprodukt (Angaben in Gew.-%):

|          |      |
|----------|------|
| WAS:     | 71,2 |
| US:      | 8,6  |
| $Na_2SO_4$: | 6,3 |
| NaCl:    | 0,1  |
| $H_2O$:  | 12,6 |
| pH-Wert: | 9,5  |

(1 %-ige Lösung, bez. auf WAS)

Farbzahl n. Kiett: 63

(5 %-ige Lösung, bez. auf WAS, 4 cm Küvette, Blaufilter Nr. 42)

Beispiel 3

Sprühneutralisation von Fettsäuremethylestersulfonsäure (MES)

Einsatz:
MES (Basis $C_{16/18}$), ṁ ca. 20 kg/h, t ca. 80°C
Natronlauge mit 48 Gew.-% NaOH, t ca. 40°C
Sprühdruck: 8,5 bar
$N_2$-Bedarf: 2 $m^3$/h

Sprühprodukt (Angaben in Gew.-%):

|           |      |
|-----------|------|
| Mono:     | 52,3 |
| Di:       | 27,7 |
| Mono+Di:  | 80,0 |
| US:       | 6,4  |
| $Na_2SO_4$: | 6,2 |
| Wasser:   | 10,3 |
| pH-Wert:  | 9,0  |

(1 %-ige Lösung, bez. auf WAS)

Farbzahl n. Klett: 305

(5 %-ige Lösung, bez. auf WAS, 4 cm Küvette, Blaufilter Nr. 42)

Beispiel 4

Sprühneutralisation von Fettalkoholethersulfonsäure (FAES)

Einsatz:

FAES (Basis $C_{12/14}$; 2 Mol EO) mit 380 ppm Dioxan,. t ca. 30°C

Natronlauge mit 50 Gew.-% NaOH, t ca. 30°C

$N_2$-Bedarf: 2m³/h

Sprühdruck: 5 bar

Sprühprodukt (Angaben in Gew.-%):

| | |
|---|---|
| WAS: | 77,2 |
| US: | 3,8 |
| $Na_2SO_4$: | 1,2 |
| $H_2O$: | 9,4 |
| Dioxan: | unter 200 ppm |
| pH-Wert: | 10,1 |

(1 %-ige Lösung bez. auf WAS)

Farbzahl nach Klett:   37

(5 %-ige Lösung, bez. auf WAS; 4 cm Küvette, Blaufilter Nr. 42)

Beispiel 5

Sprühneutralisation von Fettsäure

Einsatz:

Palmitinsäure, ṁ 60 kg/h, t 70°C

Natronlauge mit 42 Gew.-% NaOH, t 40°C

Sprühdruck: 3,5 bar

$N_2$-Bedarf: 2 m³/h

Sprühprodukt (Angaben in Gew.-%):

| | |
|---|---|
| Na-Seife: | 69,9 |
| $H_2O$: | 26,8 |
| Palmitinsäure: | 0 |
| pH-Wert: | 9,7 |

(1 %-ige Lösung bzw. Na-Seife)

Beispiel 6:

Verseifung von Fettsäuremethylestern

Einsatz:

$C_{14}$-Methylester, ṁ 30 kg/h, t 125°C

Natronlauge mit 50 Gew.-% NaOH, t 110°C

Sprühdruck: 6,5 bar

$N_2$-Bedarf: 1 m³/h

Dampf-Bedarf: 1 m³/h

Düsentemperatur (Vorwärmung 150°C)

Sprühprodukt (Angaben in Gew.-%):

| | |
|---|---|
| Na-Seife: | 86,6 |
| $H_2O$: | 13,7 |
| $C_{14}$-Methylester: | 0,1—0,2 |
| Methanol: | 2,0 |
| pH-Wert: | 9,3 |

(1 %-ige Lösung, Na-Seife)

# EP 0 319 819 B1

## Patentansprüche

1. Verfahren zur Herstellung von festen oder pastenförmigen Produkten durch Umsetzung stöchiometrischer Mengen von mehreren Reaktanden in einer schnell ablaufenden Reaktion und Versprühen der Reaktionsprodukte in einer Sprüheinrichtung, dadurch gekennzeichnet, daß man die getrennten Reaktandenströme, gegebenenfalls nach Erwärmen, jeweils mit einem gasförmigen Medium beaufschlagt, anschließend zusammenführt und schließlich durch die Sprüheinrichtung mit einer Geschwindigkeit von 0,1 bis 15 m/sec, errechnet aus den gasfreien Reaktandenströmen und bezogen auf den freien Querschnitt der Sprüheinrichtung, preßt, wobei die Massen von Reaktanden und gasförmigem Medium so bemessen sind, daß sich ein Massenverhältnis von gasförmigem Medium zu Produktstrom zwischen 0,04 und 0,3 (kg/kg) ergibt.

2. Verfahren nach Anspruch 1, dadurch gekennzeichnet, daß man Säuren mit konzentrierten wäßrigen Alkalien neutralisiert.

3. Verfahren nach Anspruch 1, dadurch gekennzeichnet, daß man Ester mit konzentrierten wäßrigen Alkalien verseift.

4. Verfahren nach einem der Ansprüche 1 bis 3 zur Herstellung fester, rieselfähiger, waschaktiver Produkte.

5. Verfahren nach einem der Ansprüche 1 bis 4 zur Herstellung von waschaktiven Alkalisulfaten bzw. Sulfonaten, dadurch gekennzeichnet, daß einer der Reaktandenströme, insbesondere der Säurestrom, eine Lösung bzw. Suspension von Puffersubstanzen enthält.

6. Verfahren nach Anspruch 5, dadurch gekennzeichnet, daß man als Puffersubstanz Natriumperborat verwendet.

7. Verfahren nach einem der Ansprüche 1 bis 6 zur Herstellung fester, rieselfähiger, waschaktiver Produkte.

8. Verfahren nach einem der Ansprüche 1 bis 7 zur Herstellung von Produkten, die frei von niedrigsiedenden Lösemitteln sind.

9. Verfahren nach einem der Ansprüche 1 bis 8 zur Herstellung von Fettalkoholethersulfaten mit einem Dioxangehalt von weniger als 200 ppm, bezogen auf Feststoffgehalt, durch Neutralisation von Schwefelsäurehalbestern von Fettalkoholethoxylaten mit wäßrigen Alkalien.

10. Verfahren nach Anspruch 1, dadurch gekennzeichnet, daß man Monochloressigsäure mit konzentrierten Alkalilaugen umsetzt.

11. Verfahren nach Anspruch 1, dadurch gekennzeichnet, daß man tertiäre Amine alkyliert.

## Revendications

1. Procédé de fabrication de produits solides ou pâteux en faisant réagir des quantités stoechiométriques de plusieurs réactifs dans la cadre d'une réaction au déroulement rapide et en pulvérisant les produits de réaction dans une installation de pulvérisation, caractérisé en ce que les flux de réactifs séparés sont mis en contact chacun, éventuellement après chauffage, avec un milieu gazeux, réunis ensuite et finalement comprimés par l'installation de pulvérisation à une vitesse de 0,1 à 15 m/sec, calculée sur base des flux de réactifs exempts de gaz et par rapport à la section libre de l'installation de pulvérisation, les masses des réactifs et du milieu gazeux étant déterminées de manière que le rapport massique entre le milieu gazeux et le flux de produit soit compris entre 0,04 et 0,3 (kg/kg).

2. Procédé selon la revendication 1, caractérisé en ce que l'on neutralise des acides au moyen d'alcali aqueux concentrés.

3. Procédé selon la revendication 1, caractérisé en ce que l'on saponifie des esters au moyen d'alcalis aqueux concentrés.

4. Procédé selon l'une des revendications 1 à 3, pour la fabrication de produits détergents-actifs, solides et capables d'écoulement.

5. Procédé selon l'une des revendications 1 à 4, pour la fabrication de sulfates ou de sulfonates de métaux alcalins détergents-actifs, caractérisé en ce que l'un des flux de réactifs, en particulier le flux d'acide, renferme une solution ou une suspension de substances tampons.

6. Procédé selon la revendication 5, caractérisé en ce que l'on utilise comme substance tampon, du perborate de sodium.

7. Procédé selon l'une des revendications 1 à 6, pour la fabrication de produits détergents-actifs, solides et capables d'écoulement.

8. Procédé selon l'une des revendications 1 à 7, pour la fabrication de produits, qui sont exempts de solvants à bas point d'ébullition.

9. Procédé selon l'une des revendications 1 à 8, pour la fabrication de sulfates d'éther d'alcool gras dont la concentration en dioxanne est inférieure à 200 ppm par rapport à la teneur en matière solide, par neutralisation de semi-esters d'acide sulfurique d'éthoxylates d'alcool gras par des alcalis aqueux.

10. Procédé selon la revendication 1, caractérisé en ce que l'on fait réagir de l'acide monochloracétique avec des lessives alcalines concentrées.

11. Procédé selon la revendication 1, caractérisé en ce que l'on alkyle des amines tertiaires.

**Claims**

1. A process for the production of solid or paste-form products by reaction of stoichiometric quantities of several reactants in a fast reaction and spraying of the reaction products in a spray unit, characterized in that a gaseous medium is added to each of the separate reactant streams, optionally after heating, the reactant streams are then combined and, finally, are forced through the spray unit at a rate of 0.1 to 15 m/ second, as calculated from the gas-free reactant streams and based on the free cross-section of the spray unit, the masses of the reactants and the gaseous medium being gauged in such a way that a mass ratio of gaseous medium to product stream of from 0.04 to 0.3 (kg/kg) is obtained.

2. A process as claimed in Claim 1, characterized in that acids are neutralized with concentrated aqueous alkalis.

3. A process as claimed in Claim 1, characterized in that esters are saponified with concentrated aqueous alkalis.

4. A process as claimed in any of Claims 1 to 3 for the production of solid, free-flowing washing-active products.

5. A process as claimed in any of Claims 1 to 4 for the production of washing-active alkali sulfates or sulfonates, characterized in that one of the reactant streams, particularly the acid stream, contains a solution or suspension of buffers.

6. A process as claimed in Claim 5, characterized in that sodium perborate is used as the buffer.

7. A process as claimed in any of Claims 1 to 6 for the production of solid, free-flowing washing-active products.

8. A process as claimed in any of Claims 1 to 7 for the production of products which are free from low-boiling solvents.

9. A process as claimed in any of Claims 1 to 8 for the production of fatty alcohol ether sulfates having a dioxane content of less than 200 ppm, based on the solids content, by neutralization of sulfuric acid semiesters of fatty alcohol ethoxylates with aqueous alkalis.

10. A process as claimed in Claim 1, characterized in that monochloroacetic acid is reacted with concentrated alkali hydroxides.

11. A process as claimed in Claim 1, characterized in that tertiary amines are alkylated.